# EUROPÄISCHE PATENTSCHRIFT

(11) **EP 1 496 865 B1**
(45) Veröffentlichungstag und Bekanntmachung des Hinweises auf die Patenterteilung: **12.07.2006**
(21) Anmeldenummer: 03730046.4
(22) Anmeldetag: 22.04.2003
(51) Int. Cl.: A61K 9/14, C07J 41/00

(54) **VERFAHREN ZUM HERSTELLEN VON KRISTALLEN, DANACH ERHÄLTLICHE KRISTALLE UND DEREN VERWENDUNG IN PHARMAZEUTISCHEN FORMULIERUNGEN**
METHOD FOR THE PRODUCTION OF CRYSTALS, CRYSTALS OBTAINED BY SAID METHOD, AND USE THEREOF IN PHARMACEUTICAL FORMULATIONS
PROCEDE POUR PRODUIRE DES CRISTAUX, CRISTAUX AINSI OBTENUS ET LEUR UTILISATION DANS DES FORMULATIONS PHARMACEUTIQUES

(30) Priorität: 23.04.2002 DE 10218109
(43) Veröffentlichungstag der Anmeldung: 19.01.2005
(73) Patentinhaber: Schering Aktiengesellschaft, 13353 Berlin (DE)
(72) Erfinder: GRAWE, Detlef, 99510 Kleinromstedt (DE); GERECKE, Hagen, 07743 Jena (DE); HÖSEL, Peter, 07745 Jena (DE); EICHARDT, Annette, 07616 Bürgel (DE); GLIESING, Sabine, 07743 Jena (DE); MÜLLER, Uwe, 07747 Jena (DE)
(74) Vertreter: Geyer, Fehners & Partner
(86) Internationale Anmeldenummer: PCT/EP2003/005102
(87) Internationale Veröffentlichungsnummer: WO 2003/090714

(56) Entgegenhaltungen:
- EP-A- 0 499 299
- EP-A- 0 648 778
- EP-A- 1 157 996
- WO-A-98/57648
- DD-A- 275 398
- US-A- 5 534 270

## Beschreibung

Die Erfindung bezieht sich auf ein Verfahren zum Herstellen von Kristallen, deren durchschnittliche Partikelgröße 3 µm bis 25 µm beträgt und deren maximale Partikelgröße 100 µm nicht überschreiten, nach diesem Verfahren erhältliche Kristalle und deren Verwendung in pharmazeutischen Formulierungen, insbesondere low dose Formulierungen.

Aus der EP 0 648 778 A2 sind 11β-Benzaldoxim-estra-4,9-dien-Derivate bekannt. Dort ist die Synthese und Reinigung dieser Verbindungen beschrieben. Ein Kristallisations- bzw. Formgebungsschritt ist dort nicht aufgeführt. Wie bei den meisten Steroiden werden sie aus einem geeigneten Lösungsmittel kristallisiert. Bei einer konventionellen Kühlungs- oder Verdrängungskristallisation entsteht allerdings ein nicht weiter definiertes grobkörniges Kristallisat. Aus der EP 1 157 996 sind Festkörperformen des Mesoprogesterins Oxim J 867, insbesondere hochreine und stabile amorphe bzw. hochkristalline formen bekannt

Für low dose Formulierungen, die den Wirkstoff nur in geringen Mengen enthalten, beispielsweise 0,1 Gew.-% bis 2 Gew.-%, werden besondere Anforderungen an die Homogenität der Wirkstoffverteilung (content uniformity, CUT) und die Dissolutionskinetik gestellt. Bei diesen low dose Formulierungen wird der in sehr geringen Mengen vorhandene Wirkstoff mit den anderen Arzneimittelbestandteilen in ganz erheblichem Maß verdünnt. Damit die Homogenität der Wirkstoffverteilung annähernd konstant bleibt, dürfen bestimmte durchschnittliche Korngrößen nicht überschritten werden, und die Streubreite darf nicht zu hoch sein. Diese maximalen Korngrößen hängen von der Dosierung und der Applikationsform ab und können statistisch ermittelt werden. Des weiteren ist bei low dose Formulierungen zu beachten, daß kleine Partikel im Magen schneller als große aufgelöst werden. Damit die Forderungen bezüglich Dissolutionskinetik erfüllt werden (in 45 Minuten mehr als 70 % Wirkstoff aufgelöst), ist es notwendig, daß bestimmte Partikelgrößen nicht überschritten werden.

Um diesen Anforderungen an die Homogenität der Wirkstoffverteilung und der Dissolutionskinetik bei low dose Formulierungen zu erreichen, werden bisher die Kristallisate nach traditioneller Technologie in einer Strahlmühle mikronisiert. Dabei werden durchschnittliche Korngrößen von 1,5 µm bis 3 µm erhalten. Es erfolgt allerdings eine enorme Vergrößerung und eine thermodynamische Aktivierung der Oberfläche der Kristalle durch partielle Amorphisierung bzw. durch erhebliche Störungen in der Gitterstruktur. Diese physikalischen Veränderungen bewirken eine chemische Destabilisierung des Wirkstoffs nicht nur in reiner Form, sonder auch und vor allem in pharmazeutischen Formulierungen.

Die Carbamatfunktion der vorstehend erwähnten 11β-Benzaldoxim-estra-4,9-dien-Derivaten wird unter Abspaltung von Ethylamin und CO₂ zum Nitril abgebaut. Die Verwendung von Mikronisaten führt somit nachweislich zu Arzneiformen, in welchen der Wirkstoff unter ICH (40°C, 70% relative Luftfeuchtigkeit) nicht ausreichend stabil ist.

Eine Absenkung des Mahldrucks beim Mikronisieren führt zwar zu einer geringfügigen Erhöhung der durchschnittlichen Partikelgröße, aber auch zu einem unerwünschten Anstieg ihrer Streubreite. Für die Funktion der Mühle ist jedoch ein bestimmter Mindestdruck unbedingt erforderlich. Eine Beeinflussung der Festkörperform in Richtung verbesserter chemischer Stabilität durch die Mikronisierparameter ist also wenn überhaupt nur sehr begrenzt möglich.

Der vorliegenden Erfindung liegt somit die Aufgabe zugrunde, ein Verfahren zum Herstellen von Kristallen bereitzustellen, das nicht die aus dem Stand der Technik bekannten Nachteile aufweist und mit dem insbesondere Kristalle erhältlich sind, die die Anforderungen an low dose Formulierungen erfüllen.

Erfindungsgemäß wird dies erreicht durch ein Verfahren zum Herstellen von Kristallen, deren durchschnittliche Partikelgröße in einem vorgegebenen Bereich liegt und deren maximale Partikelgröße einen vorgegebenen Wert nicht überschreitet, wobei eine übersättigte Lösung von 11β-Benzaldoxim-estra-4,9-dien der Formel (1) 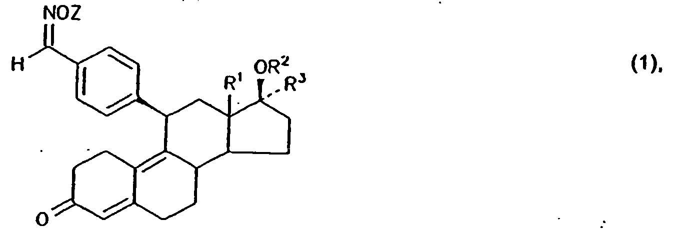 worin
R¹ ein Wasserstoffatom oder ein Alkylrest mit 1-6 Kohlenstoffatomen ist,
R² für ein Wasserstoffatom, eine Alkyl-, Aryl-, Aralkyl- oder Alkylarylgruppe mit 1-10 Kohlenstoffatomen, einen Acylrest mit 1-10 Kohlenstoffatomen oder einen Rest -CONHR⁴ oder -COOR⁴ steht, wobei R⁴ ein Wasserstoffatom, einen Alkyl-, Aryl-, Aralkyl- oder Alkylarylrest mit 1-10 Kohlenstoffatomen bedeutet,
R³ ein Wasserstoffatom, eine Alkyl-, Aryl-, Aralkyl- oder Alkylarylgruppe mit 1-10 Kohlenstoffatomen, einen Rest -(CH₂)ₙ-CH₂X, wobei n=0, 1 oder 2 ist, X für ein Wasserstoffatom, für einen Alkyl-, Aryl-, Aralkyl- oder Alkylarylrest mit 1-10 Kohlenstoffatomen, für ein Fluor-, Chlor-, Brom- oder lodatom, für eine Cyano-, Azido- oder Rhodanogruppe, für einen Rest OR⁵ oder SR⁵ steht, wobei R⁵ ein Wasserstoffatom, ein Alkyl-, Aryl-, Aralkyl- oder Alkylarylrest mit 1-10 Kohlenstoffatomen oder ein Acylrest mit 1-10 Kohlenstoffatomen ist,
einen Rest OR⁵, wobei R⁵ die oben angegebene Bedeutung hat,
einen Rest -(CH₂)ₒ-CH=CH(CH₂)ₚ-R⁶, wobei o=0, 1, 2 oder 3 und p=0, 1 oder 2 ist und R⁶ für ein Wasserstoffatom, eine Alkyl-, Aryl-, Aralkyl- oder Alkylarylgruppe mit 1-10 Kohlenstoffatomen, eine Hydroxylgruppe, eine Alkoxygruppe oder Acyloxygruppe mit 1-10 Kohlenstoffatomen steht,
einen Rest -(CH₂)_{q}C=CR⁷, wobei q=0, 1 oder 2 und R⁷ ein Wasserstoffatom, ein Fluor-, Chlor-, Brom- oder lodatom, ein Alkyl-, Aryl-, Aralkyl- oder Alkylarylrest mit 1-10 Kohlenstoffatomen oder ein Acylrest mit 1-10 Kohlenstoffatomen ist, bedeutet,
Z für ein Wasserstoffatom, einen Alkyl-, Aryl-, Aralkyl- oder Alkylarylrest mit 1-10 Kohlenstoffatomen, einen Aclrest mit 1-10 Kohlenstoffatomen, einen Rest -CONHR⁴ oder -COOR⁴, wobei R⁴ ein Wasserstoffatom, einen Alkyl-, Aryl-, Aralkyl- oder Alkylarylrest mit 1-10 Kohlenstoffatomen bedeutet,
oder für ein Alkali- oder Erdalkalimetallatom steht, sowie deren pharmazeutisch annehmbaren Salze,
während der Kristallisation einem Naßmahlen mittels einer Vorrichtung zum Naßmahlen unterzogen wird, wodurch eine Primärkomsuspension erhalten wird.

Mit dem erfindungsgemäßen Verfahren ist es in überraschender Weise möglich, Kristalle zu erhalten, die ausreichend stabil sind und hinsichtlich der Parameter ihrer Partikelgrößenverteilung den pharmazeutischen Anforderungen bezüglich Homogenität der Wirkstoffverteilung (CUT) und Dissolutionskinetik für low dose Formulierungen eingestellt und somit gerecht werden können. Des weiteren kann eine für die jeweilige Dosis geeignete Korngrößenverteilung mit hoher Zielgenauigkeit und Reproduzierbarkeit hergestellt werden. Ferner kann das erfindungsgemäße Verfahren in einfacher, schneller und kostengünstiger Weise durchgeführt werden.

In einer bevorzugten Ausführungsform des erfindungsgemäßen Verfahrens wird die Verbindung 11β-{4-[(Ethylaminocarbonyl)oximinomethyl]phenyl}-17β-methoxy-17α-methoxymethyl-estra-4,9-dien-3-on- eingesetzt. Bei Verwendung dieser Verbindung im erfindungsgemäßen Verfahren werden die vorstehend beschriebenen Vorteile in besonders günstiger Weise erreicht.

Die Erfindung wird nachstehend unter Bezugnahme auf die Figuren näher erläutert, wobei
- Fig. 1: das IR-Spektrum von 11β-{4-[Ethylaminocarbonyl)oximinomethyl]phenyl}-17β-methoxy-17α-methoxymethyl-estra-4,9-dien-3on(J956) zeigt;
- Fig. 2: die Nitrilbildung von J956 als Funktion der Korngröße darstellt, und
- Fig. 3 und 4: die Entwicklung der Korngröße beim erfindungsgemäßen Kristallisations-verfahren zeigen.

Die durchschnittliche Partikelgröße beträgt 3 µm bis 25 µm, insbesondere 7 µm bis 15 µm. Die maximale Partikelgröße überschreitet nicht 100 µm, insbesondere 80 µm. Der Ausdruck "maximale Partikelgröße" bedeutet dabei, daß kein Teilchen größer als der angegebene Wert ist. Innerhalb dieser Grenzen der durchschnittlichen Partikelgröße und der maximalen Partikelgröße ist es in günstiger Weise möglich, die Partikelgrößenverteilung so zu wählen, daß sie den pharmazeutischen Anforderungen bezüglich CUT und Dissolutionskinetik für low dose Formulierungen entspricht.

Im erfindungsgemäßen Verfahren wird eine übersättigte Lösung einer Verbindung der Formel (1) eingesetzt. Die Lösung enthält als Gelöstes die Verbindung der Formel (1), die in einem Lösungsmittel dafür gelöst ist. Als Lösungsmittel werden auch Gemische verschiedener Lösungsmittel verstanden. Eine im erfindungsgemäßen Verfahren eingesetzte übersättigte Lösung, die beispielsweise durch Unterkühlung hergestellt werden kann, enthält mehr gelösten Stoff als sie in ihrem thermischen Gleichgewicht aufweisen dürfte. Es können im erfindungsgemäßen Verfahren übersättigte Lösungen eingesetzt werden, in denen Keime spontan gebildet werden.

In einer bevorzugten Ausführungsform des erfindungsgemäßen Verfahrens enthält die übersättigte Lösung 10 Gew.-% bis 30 Gew.-%, insbesondere etwa 20 Gew.-%, bezogen auf die übersättigte Lösung, der Verbindung der Formel (1). Mit diesen übersättigten Lösungen können die vorstehend beschriebenen Vorteile des erfindungsgemäßen Verfahrens in besonders günstiger Weise erreicht werden.

Vorzugsweise ist das Lösungsmittel für die übersättigte Lösung Ethylacetat, das sich als besonders günstig für die Herstellung übersättigter Lösungen der Verbindungen der Formel (1) erwiesen hat.

Die Herstellung der übersättigten Lösungen kann in üblicher Weise erfolgen. Vorzugsweise wird die übersättigte Lösung hergestellt durch Auflösen der Verbindung der Formel (1) in einem Lösungsmittel bei einer Temperatur unterhalb des Siedepunkts und nachfolgendem Abkühlen auf eine Temperatur oberhalb des Gefrierpunkts der Lösung. Wird das für das erfindungsgemäße Verfahren bevorzugte Ethylacetat als Lösungsmittel für die übersättigte Lösung eingesetzt, kann das Erwärmen auf beispielsweise etwa 70°C erfolgen, bis die Verbindung der Formel (1) im Ethylacetat klar gelöst ist. Das Abkühlen kann während 10 Minuten bis 1 Stunde, insbesondere 15 Minuten bis 30 Minuten, auf etwa 50°C bis 10°C, vorzugsweise 30°C bis 35°C, erfolgen. Der Fachmann kann durch einfache Tests aufgrund vorstehender Angaben die Parameter zur Herstellung einer übersättigten Lösung mit einem anderen Lösungsmittel als Ethylacetat ohne weiteres ermitteln.

Günstigerweise wird die Kristallisation in einem Gefäß durchgeführt, das einen Rührer aufweist. Beispiel dafür sind die für technische Anwendungen an sich bekannten Kristallisatoren.

Im erfindungsgemäßen Verfahren erfolgt während der Kristallisation ein Naßmahlen mittels einer Vorrichtung zum Naßmahlen. Die Kristallisation aus der übersättigten Lösung kann einsetzen, nachdem mit dem Naßmahlen begonnen wurde. Geeignete Vorrichtungen für den Schritt des Naßmahlens sind Dispergiervverkzeuge und Homogenisatoren, wie Rotor-Stator-Werkzeuge, Rührwerksmühlen, Walzenstühle und Kolloidmühlen.

Die erfindungsgemäße Herstellung der Kristalle erfolgt, wie bereits vorstehend beschrieben, durch Kristallisation aus einem Lösungsmittel oder Lösungsmittelgemisch, vorzugsweise aus einer durch Abkühlung erzeugten übersättigten Ethylacetatlösung, indem in der Startphase der Kristallisation, entweder kurz nachdem die Kristallisation begonnen hat oder bevor sie begonnen hat, zusätzlich zum konventionellen Rührwerk ein Naßmahlen mittels einer Vorrichtung zum Naßmahlen, insbesondere eines Rotor-Stator-Werkzeugs oder einer Kolloidmühle, durchgeführt wird. Diese Vorrichtung zum Naßmahlen kann direkt als zusätzliches Rührwerk im Kristallisationsgefäß oder in einer Umlaufschleife des Kristallisators eingesetzt werden. Wird ein Rotor-Stator-Werkzeug verwendet, so kann die Rotorumfangsgeschwindigkeit 10 m/s bis 50 m/s, vorzugsweise 20 m/s bis 40 m/s betragen. Durch den durch das Naßmahlen, insbesondere den Rotor-Stator, bewirkten zusätzlichen Energieeintrag wird eine sehr hohe sekundäre Keimbildungsrate erzeugt und dadurch das Kristallwachstum stark eingeschränkt. Zusätzlich werden sich evtl. bildende Agglomerate im engen Scherspalt zerschlagen. Somit wird ein feines Primärkom erzeugt, dessen durchschnittliche Partikelgröße je nach eingestellter Übersättigung und Rotorumfangsgeschwindigkeit zwischen 3 µm und 5 µm beträgt und dessen maximale Partikelgröße 25 µm bis 60 µm nicht übersteigt. Diese Partikelparameter können für low dose Formulierungen bereits ausreichend sein.

Um entsprechend den pharmazeutischen Anforderungen auch gröbere Kömungen mit definierter Partikelgrößenverteilung mit entsprechender Zielgenauigkeit und hoher Reproduzierbarkeit herstellen zu können, wird die Primärkomsuspension vorzugsweise einem oszillatorischen Temperaturprofil unterworfen. Hierzu wird die erzeugte feine Primärkomsuspension auf eine Temperatur Tₘₐₓ unterhalb der Löslichkeitsgrenze der Primärkömer in der Suspension erwärmt und nachfolgend langsam auf eine Temperatur Tₘᵢₙ, die oberhalb des Gefrierpunkts der Suspension liegt, abgekühlt. Beim Aufwärmvorgang wird die Feinkomfraktion der Primärkomsuspension aufgelöst und bei einem anschließenden Kühlvorgang auf die vorhandene Grobkomfraktion aufkristallisiert. Hierdurch ergibt sich eine definierte Verschiebung der Partikelgrößenverteilung zum gröberen Bereich. Vorzugsweise wird Tₘₐₓ so gewählt, daß 10 Gew.-% bis 90 Gew.-%, insbesondere 20 Gew.-% bis 50 Gew.-%, ganz besonders etwa 30 Gew.-% der Primärkömer im Lösungsmittel aufgelöst werden. Der Anteil der aufzulösenden Menge der Primärkömer wird in Abhängigkeit von der vorgegebenen Kömung gewählt, die wiederum durch die Art der low dose Formulierung bestimmt ist. Wird ein hoher Anteil der Primärkömer aufgelöst, wird eine grobere Körnung erhalten.

In einer bevorzugten Ausführungsform des erfindungsgemäßen Verfahrens wird Tₘᵢₙ so gewählt, daß die aufgelösten Primärkörner im wesentlichen wieder kristallisieren. Günstigerweise sollten, um die Verluste an Verbindungen der Formel (1) gering zu halten, nahezu alle der aufgelösten Primärkömer an den noch verbleibenden Primärkömem kristallisieren.

Vorzugsweise erfolgt das Abkühlen von Tₘₐₓ auf Tₘᵢₙ während 1 Minute bis 10 Stunden, insbesondere während 0,5 Stunden bis 2 Stunden.

Die Abkühlflanke des Temperaturprofils sollte dabei so gesteuert werden, daß die erneute Keimbildung möglichst gering gehalten wird. Die Schrittweite dieser Vergröberung ist abhängig von dem im Heizzyklus aufgelösten Mengenanteil des Kristallisates, welcher wiederum von Lage der beiden Temperaturen Tₘₐₓ und Tₘᵢₙ in bezug auf die Löslichkeitsgrenze und von der Feststoffkonzentration der Suspension bestimmt ist. Dieser Heiz-Kühlzyklus kann so oft wiederholt werden, vorzugsweise 1 bis 10 mal, bis die gewünschte Partikelgrößenverteilung erreicht wird. Steuerparameter sind dabei Tₘₐₓ, Tₘᵢₙ und die Anzahl der Zyklen. Je geringer die gewünschte Vergröberung, um so geringer sollte Tₘₐₓ gewählt werden. Somit kann man sich in kleinen Schritten der gewünschten Endkömung annähem. Der Verlauf des aufgelösten Anteils des Kristallisates in den Heizperioden wird dabei so dimensioniert, daß der maximale Partikeldurchmesser nur noch in sehr geringem Maße zunimmt und die Vergröberung im Bereich der feineren Partikeln stattfindet. So wird beispielsweise bei Auflösung und Rekristallisieren von 40% der aus einer 20 Gew%-igen Essigesterlösung auskristallisierten J956 der durchschnittliche Partikeldurchmesser (X50) von 4.9 µm auf 7.8 µm erhöht, während die Vergrößerung der maximalen Korngröße (X100) kaum meßbar ist. Dies bedeutet, die Partikelgrößenverteilung wird bei Wachstum ihres Durchschnittwertes (X50) deutlich enger. Bezüglich der pharmazeutischen Verwendung, insbesondere für die Erzielung entsprechender CUT-Werte und Dissolutionseigenschaften, ist dieser Effekt besonders vorteilhaft .

Nach der Durchführung des oszillatorischen Temperaturprofils kann die erhaltene Kristallsuspension filtriert und mit einem Lösungsmittel gewaschen werden, indem die Verbindung der Formel (1) nur in geringen Mengen von beispielsweise weniger als 1 Gew.-% löslich ist. Beispiele solcher Lösungsmittel sind Methyl-tert.-butylether, Hexan, Heptan, Wasser oder Gemische von zwei oder mehreren dieser. Dadurch wird beim anschließenden Trocknungsprozeß, der vorteilhafterweise direkt in der Filtrationseinheit durch ein Trocknungsgas oder im Vakuum erfolgt, eine Brückenbildung und Agglomeration der Partikel vermieden.

Die Trocknung kann durch Konvektions- oder Vakuumtrocknung in ruhender oder bewegter Schüttung erfolgen.

Wenn eine konventionelle Filtration und Trocknung schwer möglich ist und zu einer Beeinträchtigung der bei der Kristallisation erzeugten Partikelgrößenverteilung führt, wie beispielsweise im Falle sehr feiner Körnungen, kann alternativ der filtrierte und gewaschene Filterkuchen mit einer Suspendierflüssigkeit mit sehr geringer Löslichkeit für die Verbindung der Formel (1), beispielsweise weniger als 1 Gew.-%, vorzugsweise Wasser, aufgeschlämmt werden. Die erhaltene Suspension kann über Sprühtrocknung in die getrocknete feste Form der Verbindung der Formel (1) überführt werden.

Gegenstand der vorliegenden Erfindung sind weiterhin Kristalle der Verbindung der Formel (1), die nach dem erfindungsgemäßen Verfahren erhältlich sind. Zur Durchführung des Verfahrens wird auf die vorstehenden Ausführungen, in denen das erfindungsgemäße Verfahren im Detail beschrieben wurde, verwiesen.

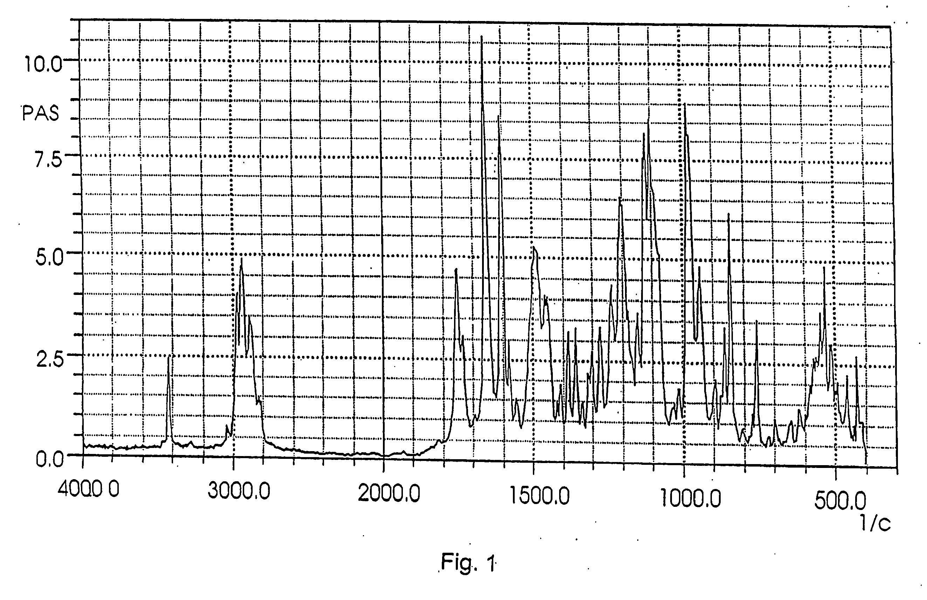

Wird als Verbindung der Formel (1) das Steroid 11β-{4-[(Ethylaminocarbonyl)oximinomethyl]phenyl}-17β-methoxy-17α-methoxymethyl-estra-4,9-dien-3-on (im weiteren als J956 bezeichnet) im erfindungsgemäßen Verfahren verwendet, so werden die in der nachfolgenden Tabelle 1 angegebenen Röntgenpulverdiffraktometrie-Daten sowie das in Fig. 1 angegebene IR-Spektrum erhalten.

| d_{obs} (Å) | l_{obs} | dₜₕₑₒ (Å) | Iₜₕₑₒ | h k l |
|---|---|---|---|---|
| nicht ermittelt* | - | 12.992 | 10.3 | 2 0 0 |
| 10.34 | 32.7 | 10.322 | 97.2 | 0 0 1 |
| 9.56 | 18.7 | 9.560 | 51.1 | 1 1 0 |
| 9.30 | 0.9 | 9.302 | 4.7 | -2 0 1 |
| 7.25 | 4.3 | 7.243 | 13.6 | 2 0 1 |
| 6.63 | 14.5 | 6.624 | 42.3 | 3 1 0 |
| 6.26 | 9.6 | 6.253 | 31.8 | -4 0 1 |
| 6.14 | 26.4 | 6.137 | 54.3 | -3 1 1 |
| 5.27 | 14.5 | 5.273 | 33.1 | -2 0 2 |
| 5.14** | 100.0 | 5.143 | 50.3 | 3 1 1 |
| | | 5.140 | 100.0 | 0 2 0 |
| 4.78 | 10.9 | 4.780 | 21.7 | 2 2 0 |
| 4.73 | 7.3 | 4.728 | 12.5 | -1 1 2 |
| 4.64** | 43.1 | 4.638 | 47.0 | 5 1 0 |
| | | 4.637 | 53.1 | -5 1 1 |
| 4.60 | 15.7 | 4.601 | 28.3 | 0 2 1 |
| 4.50 | 10.1 | 4.499 | 14.3 | -2 2 1 |
| 4.43 | 4.6 | 4.429 | 11.2 | 2 0 2 |
| 4.19 | 18.9 | 4.192 | 36.1 | 2 2 1 |
| 4.03 | 8.2 | 4.031 | 15.5 | 4 2 0 |
| 3.97 | 3.5 | 3.971 | 5.8 | -4 2 1 |
| 3.92 | 6.0 | 3.915 | 12.8 | 5 1 1 |
| 3.74 | 4.3 | 3.737 | 9.6 | 3 1 2 |
| 3.63 | 2.8 | 3.622 | 6.5 | 4 0 2 |
| 3.57 | 4.8 | 3.574 | 8.7 | -7 1 1 |
| 3.45 | 4.0 | 3.449 | 7.1 | -4 2 2 |
| 3.40 | 11.5 | 3.398 | 15.0 | 1 3 0 |
| 3.35 | 10.8 | 3.358 | 12.8 | 2 2 2 |
| 3.26 | 6.6 | 3.259 | 5.8 | -1 3 1 |
| 3.19 | 4.3 | 3.196 | 5.2 | 1 3 1 |
| 2.97 | 7.1 | 2.968 | 8.7 | 3 3 1 |

Von der Verbindung J956 sind zwei Kristallformen bekannt, wobei aber nur eine pharmazeutisch relevant ist. Diese pharmazeutisch relevante Kristallform des J956 wird durch das erfindungsgemäße Verfahren erhalten und die Röntgenpulverdiffraktometrie-Daten sind in der Tabelle 1 angegeben. Wie aus einem Vergleich der theoretischen mit den beobachteten d-Werten gesehen werden kann, liegen die Abweichungen im Bereich von weniger als 1%.

Die vorliegende Erfindung betrifft ferner pharmazeutische Formulierungen, die die nach dem erfindungsgemäßen Verfahren erhältlichen Kristalle der Verbindungen der Formel (1) aufweisen. Als pharmazeutisch wirksame Arzneiformen, insbesondere für die perorale Anwendung werden beispielsweise Hartgelatinekapseln oder Tabletten mit und ohne Überzug verwendet. Die mit den mikrokristallinen Steroid der Formel (1) hergestellten Arzneiformen dürfen nicht die chemische und kristalline Stabilität der Mikrokristalle beeinträchtigen. Dies kann dadurch erreicht werden.
- daß die Arzneiformen einen Lichtschutz beinhalten z.B. durch gefärbte Kapselhüllen oder Aufbringen eines gefärbten Überzuges;
- daß oberflächenvergrößemde Hilfsstoffe, wie hochdisperses Siliciumdioxid, nicht eingesetzt werden;
- daß möglichst keine oder nur Wasser als Lösungsmittel oder Hilfsstoff zum Einsatz kommen, und/oder
- daß der Wassergehalt der Arzneiform durch gute Trocknung niedrig gehalten wird.

Ein Beispiel einer geeigneten Kapselrezeptur ist in Tabelle 2 angegeben:

**Tab 2: Zusammensetzung einer geeigneten Kapselrezeptur der Dosierung 1 mg J956**

| **Substanz** | **Menge** |
|---|---|
| J956, mikrokristallin | 1,000 mg |
| mikrokristalline Cellulose | 102,480 mg |
| Magnesiumstearat | 0,520 mg |
| Hartgelatinekapsel, Größe 3 | 1 Stück |
| Kapselfüllmasse | 104,000 mg |

In Tabelle 3 ist ein Beispiel einer geeigneten Tablettenrezeptur angegeben:

**Tab 3: Zusammensetzung einer geeigneten Tablettenrezeptur der Dosierung 1 mg J956**

| **Kern:** | |
|---|---|
| J956, mikrokristallin | 1,00 mg |
| Laktose-Monohydrat | 33,8 mg |
| Maisstärke | 18,0 mg |
| Maltodextrin (10 % in Wasser) | 6,0 mg |
| Na-Carboxymethylstärke | 0,6 mg |
| Glycerol-Monobehenat | 0,6 mg |

| **Hülle:** | |
|---|---|
| Hydroxypropylmethylcellulose | 1.125 mg |
| Talkum | 0.225 mg |
| Titandioxid | 0.625 mg |
| Eisenoxid, Pigment gelb | 0.020 mg |
| Eisenoxid, Pigment rot | 0.005 mg |

Ein wesentliches Ergebnis der Erfindung besteht darin, daß Mikrokristalle der Steroide der Formel (1) erhältlich sind, die chemisch deutlich stabiler sind als bisher bekannte Mikronisate, da sie zum einen eine geringere spezifische Oberfläche und zum anderen eine durch den erfindungsgemäßen Kristallisationsprozeß ungestörte und hochkristalline Oberfläche aufweisen.

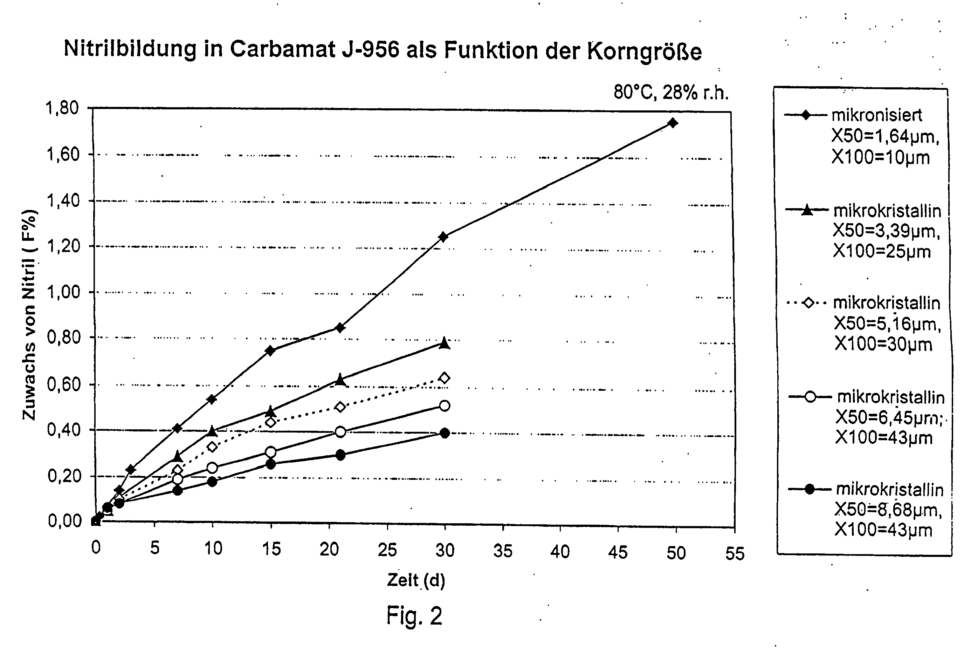

In Fig. 2 ist die Stabilität der Mikrokristalle im Vergleich zu Mikronisaten bezüglich der Nitrilbildung unter thermischem Streß (80%, 28% relative Luftfeuchtigkeit) dargestellt. Dabei zeigen die Mikrokristalle mit zunehmender Korngröße im Vergleich zu einem Mikronisat eine deutlich verbesserte Stabilität, die sich in einer verminderten Nitrilbildung darstellt.

Ein weiteres Ergebnis ist, daß die nach dem erfindungsgemäßen Verfahren erhältlichen Mikrokristalle der Steroide der Formel (1) bezüglich ihrer Korngrößenverteilung und Löslichkeitseigenschaften den pharmazeutischen Anforderungen der Arzneifertigware bezüglich CUT und Dissolution entsprechen.

Am Beispiel der 1mg Kapsel und 1 mg Tablette (vergleiche vorstehend) konnte gezeigt werden, daß die erreichten Werte denen bei Verwendung von mikronisiertem Festkörper nicht nachstehen (Tab. 4, Tab. 5).

**Tab 4: J956: Freisetzung aus 1mg Kapseln mit mikrokistallinem Festkörper im vgl. zu Kapseln mit mikronisiertem Wirkstoff**

| Testmedium: 0.3% SDS in Wasser, Paddle, 100 U/min | | | | | | |
|---|---|---|---|---|---|---|
| Korndurchmesser (µm) | | Freisetzung (%) | | | | |
| X50 | X100 | 0 min | 10 min | 20 min | 30 min | 45 min |
| 3,4 | 25 | 0 | 90,7 | 97,3 | 98,1 | 99,9 |
| 5,2 | 30 | 0 | 89,8 | 93,5 | 93,4 | 95,6 |
| 6,6 | 43 | 0 | 93,2 | 95,9 | 96,7 | 96,8 |
| 8,7 | 43 | 0 | 93,5 | 96,7 | 98,5 | 99,7 |
| 14,1 | 87 | 0 | 90,2 | 95,3 | 96,0 | 96,3 |
| Mikronisat | | 0 | 92,1 | 94,3 | 94,6 | 94,9 |

**Tab 5: J956: Schwankungsbreite der CUT-Werte 1 mg Kapsel mit mikrokristallinem Festkörper im Vergleich zu Kapseln mit mikronisiertem Wirkstoff**

| Korndurchmesser (µm) | | | |
|---|---|---|---|
| X50 | X100 | Konfidenz intervall(%) | RSD(%) |
| 3,4 | 25 | 2,23 | 3,56 |
| 5,2 | 30 | 1,20 | 2,08 |
| 6,6 | 43 | 1,08 | 1,57 |
| 8,7 | 43 | 0,93 | 1,38 |
| 14,1 | 87 | 1,77 | 2,50 |
| Mikronisat | | 1,72 | 2,56 |

**Tab 6: J956: Freisetzung aus 1 mg Tablette mit mikrokistallinem Festkörper im vgl. zu Tabletten mikronisiertem Wirkstoff**

| Testmedium: 0.3% SDS in Wasser, Paddle, 100 U/min | | | | | | |
|---|---|---|---|---|---|---|
| Korndurchmesser (µm) | | Freisetzung (%) | | | | |
| X50 | X100 | 0 min | 10 min | 20 min | 30 min | 45 min |
| 10,6 | 73 | 0 | 73,7 | 90,3 | 91,85 | 96,6 |
| Mikronisat | | 0 | 92,1 | 94,3 | 94,6 | 94,9 |

**Tab 7: J956: Schwankungsbreite der CUT-Werte 1 mg Tablette mit mikrokristallinem Festkörper im Vergleich zur Tablette mit mikronisiertem Wirkstoff**

| Korndurchmesser (µm) | | | |
|---|---|---|---|
| X50 | X100 | Konfidenz intervall(%) | RSD(%) |
| 10,6 | 73 | 1,16 | 1,70 |
| Mikronisat | | 1,72 | 2,56 |

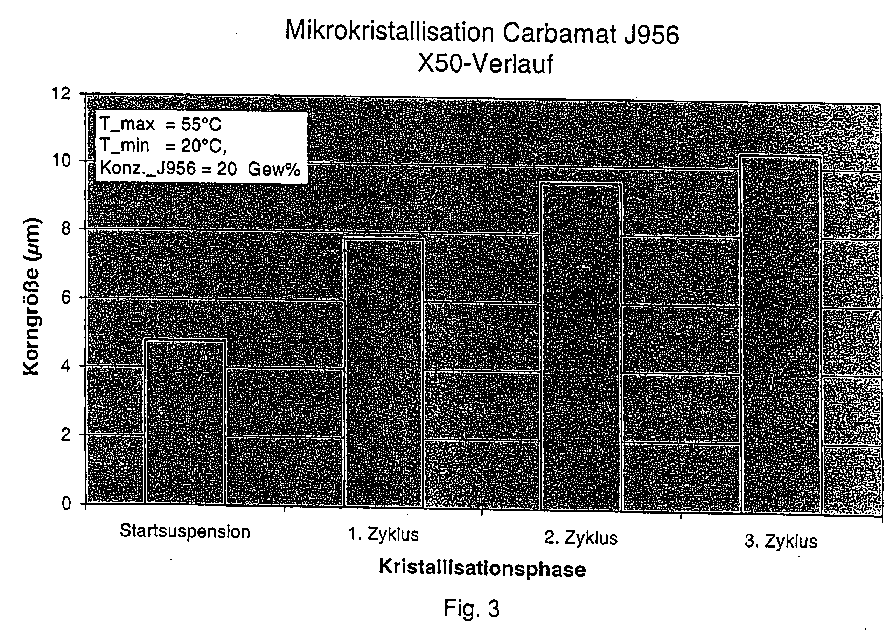

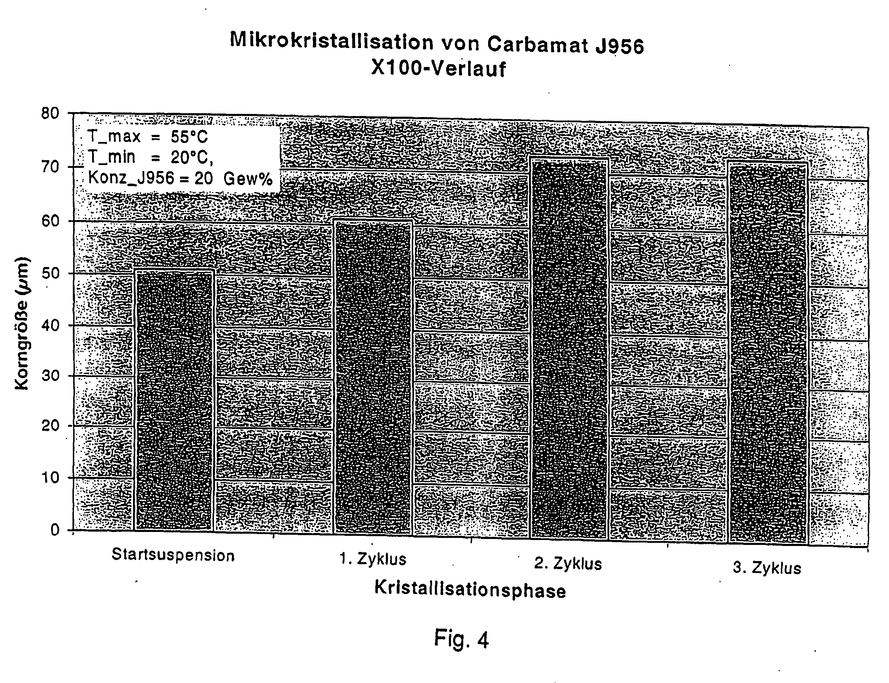

Ein weiteres wichtiges Resultat ist, daß mit dem erfindungsgemäßen Verfahren zielgenau und mit hoher Reproduzierbarkeit die pharmazeutisch erforderliche Korngrößenverteilung der Steroide der Formel (1) erzeugt werden kann. In Fig. 3 und 4 ist die Entwicklung der Korngröße im Kristallisationsverfahren dargestellt. Dabei ist von Vorteil, daß sich die Streuung der Partikelgrößenverteilung deutlich vermindert und trotz Vervielfachung der durchschnittlichen Korngröße die maximale Korngröße deutlich weniger zunimmt. Dies unterstützt die Erzielung guter CUT-Werte auch in low dose Formulierungen.

Weiter wurde erreicht, daß die in Suspension erzeugte Korngrößenverteilung auch im getrockneten Festkörper erhalten bleibt.

**Tab. 8: Korngrößenverteilung vor und nach Trocknung**

| | X10 | X50 | X90 | X100 |
|---|---|---|---|---|
| Suspension* | 2,62 | 10,4 | 24 | 73 |
| nach Trocknung auf Filter | 2,7 | 10,61 | 24 | 73 |
| | | | | |

| | X10 | X50 | X90 | X100 |
|---|---|---|---|---|
| Suspension** | 2,11 | 8,6 | 19 | 51 |
| nach Sprühtrocknung | 2,25 | 8,03 | 17 | 43 |

| | | | | |
|---|---|---|---|---|
| *) Suspension J956 in Ethylacetat mit 14 Gew.-% Mikrokristalle J956 | | | | |
| **)Suspension J956 in Wasser/Ehanol (90/10 w/w) mit 10 Gew.-% Mikrokristalle J956 | | | | |

Schließlich wurde eine pharmazeutische Formulierung gefunden, die unter Verwendung der erfindungsgemäßen Mikrokristatle hergestellt nach dem erfindungsgemäßen Verfahren eine chemisch stabile und pharmazeutisch wirksame Arzneiform darstellt.

Arzneiformen mit den erfindungsgemäßen Mikrokristallen der Steroide der Formel (1) können auf folgenden Gebieten vorteilhaft eingesetzt werden: Steroide der Formel (1), insbesondere J956, sind eine antigestagen wirkende Substanz, die bei gleicher Aktivität wie RU 486 (Mifepriston) am Progesteron-Rezeptor eine im Vergleich mit RU 8486 deutlich reduzierte antiglucocortikoide Aktivität besitzt. J956 wird als Mesoprogestin bezeichnet, wobei es als Verbindungen definiert ist, die in vivo sowohl agonistische als auch antagonistische Aktivität am Progesteron-Rezeptor (PR) aufweist. Entsprechende funktionale Zustände können mit Gestagen und Antigestagen nicht erreicht werden. Das J956 eignet sich insbesondere für folgende Verwendungen: Es kann gegebenenfalls zusammen mit einem Estrogen, zur Herstellung eines Arzneimittels zur weiblichen Kontrazeption verwendet werden, und es kann zur Behandlung und Verhinderung von benignen hormonabhängigen gynäkologischen Störungen, wie zur Behandlung von gynäkologischen Störungen, wie Endometriose, uterinen Fibroiden, postoperativen peritonealen Adhäsionen, dysfunktionaler Blutung (Metrorrhagie, Menorrhagie) und Dysmenorrhöe, sowie zur Verhinderung von gynäkologischen Störungen; wie postoperativen, peritonealen Adhäsionen, dysfunktionaler Uterusblutung (Metrorrhagie, Menorrhagie) und Dysmenorrhöe, verwendet werden. Die tägliche Dosis des Mesoprogestins kann 0,5 mg bis 100 mg, vorzugsweise 5,0 mg bis 50 mg und am stärksten bevorzugt 10 mg bis 25 mg betragen. J956 kann ebenso als pharmazeutischer Bestandteil zur Herstellung eines Arzneimittels gegebenenfalls zusammen mit einem Estrogen in der Hormon-Replacement-Therapy (HRT) und zur Behandlung von Hormonmangel und Symptomen von Hormon-Irregularität eingesetzt werden.

Zur Ermittlung der experimentellen Daten wurden folgende Meßverfahren eingesetzt:
Röntgenpulverdiffraktometrie (X-Ray Powder Diffraction; XRPD):
   Die Daten wurden mit einem STOE Powder Diffraktometer STADIP mit Germaniummonochromatischer CuKα₁-Strahlung (λ = 1.540598 Å) - zwischen 3° ≤ 2Θ ≤ 35° ermittelt.
IR-Spektroskopie:
   Verwendet wurde ein NICOLET 20 SXB mit Photoacoustic Detector MTEC (KBr, 8t, 90
   Sekunden).
Korngrößenverteilung:
   Sympatec HELOS (H0445), Trockendispergiersystem (RODOS), Druck 2 bar.
HPLC:
   Die Reinheitsbestimmung erfolgte nach folgender Methode:
      Säule: Hypersil ODS, 250 x 4 mm; 5 µm
      Eluent: Acetonitril-Tetrahydofuran-Gemisch (3:1) / Wasser = 4 / 6
      Fluß: 1 ml / min
      Detektion UV (299 nm)
      Auswertung: 100 %-Flächennormalisierung
Headspace für Restlösungsmittel:
   GC-Autosystem mit HS40 Perkin Elmer, Säule DB-Wax, 30 m x 0,23 mm, FID.
Wasserbestimmung erfolgte nach Karl Fischer
Content Uniformity Test
   Gehaltsbestimmung entsprechend USP/Ph. Eur. an Einzelkapseln nach Ausspülen durch HPLC mit externer Kalibrierung
   Säule: LiChrospher 5 µ RP-18 encapped, 150 x 3 mm
   Eluent: Acetonitril / Wasser = 45 / 55
   Fluß: 1 ml / min
   Detektion UV (272 nm)
Wirkstofffreisetzung
   Wirkstofffreisetzung in 1000 mL Wasser mit 0,3 % Natriumdodecylsulfat, 100 U/min
   Gehaltsbestimmung durch HPLC mit extemer Kalibrierung
   Säule: LiChrospher 5 µ RP-18 encapped, 150 x 3 mm
   Eluent: Acetonitril / Wasser = 45 / 55
   Fluß: 1 ml / min
   Detektion UV (272 nm)

Die nachfolgenden Beispiele dienen zur Erläuterung der Erfindung ohne sie aber darauf zu beschränken.

### Beispiel 1

In einem Glasreaktor mit Ankerrührer und Heiz/Kühldoppelmantel werden 250 g Carbamat J956 in 1100 ml Ethylacetat bei 70°C klar gelöst. Die Lösung wird innerhalb von 30 min auf 35°C gekühlt. Es wird ein Rotor-Stator Dispergierwerkzeug mit 8000-13000 U/min im Umlaufbetrieb eingesetzt. Nach 2-5 min setzt Kristallisation ein. Der Ultra Turrax wird noch weitere 10 min betrieben und dann abgestellt.
Die erhaltene Startsuspension wird auf 55°C aufgeheizt und anschließend innerhalb von 1h 20 min auf 20°C gekühlt. Dieser Prozess wird noch zweimal wiederholt.
Anschließend wird über eine Fritte filtriert und mit 500 ml kaltem MtBE gewaschen.
Der Filterkuchen wird mit Luft trockengesaugt.

Es wurden Mikrokristalle mit folgender Partikelgrößenverteilung erhalten:

| | Partikelgröße(µm) |
|---|---|
| X10 | 2,62 |
| X50 | 10,4 |
| X90 | 23 |
| X100 | 73 |

| | |
|---|---|
| Restlösemittel: 0,016% MtBE, 0,24% Ethylazetat | |

### Beispiel 2

In einem Sulfierkolben mit Blattrührer und thermostatisiertem Heiz/Kühlbad werden 50 g J956 in 200 g Ethylacetat bei 70°C klar gelöst. Die Lösung wird innerhalb von 15 min auf 35°C gekühlt. Es wird ein Rotor-Stator Dispergierwerkzeug (Ultra Turrax) eingebracht und mit einer Drehzahl von 12000-16000 U/min betrieben. Nach 2 min setzt Kristallisation ein. Der Ultra Turrax wird noch weitere 10 min betrieben und dann abgestellt.
Die erhaltene Startsuspension wird auf 50°C aufgeheizt und anschließend innerhalb von 1h auf 20°C gekühlt. Dieser Prozess wird noch zweimal wiederholt.
Anschließend wird die Suspension über eine Fritte filtriert und mit 100 ml MtBE gewaschen. Der Filterkuchen wird mit 1000 ml Wasser sehr gründlich gewaschen und anschließend in 300 g Wasser aufgeschlämmt. Die Suspension wird unter folgenden Bedingungen in einem Laborsprühtrockner mit Zweistoffdüse (QVF/Yamato) sprühgetrocknet

| | |
|---|---|
| Trocknungsgas_Eintrittstemperatur: | 170°C |
| Trocknungsgas_Austrittstemperatur: | 60°C |
| Durchsatz Trocknungsgas : | 0.23 m³/min |
| Sprühdüse(d= 2 mm) : | 2.5 bar |
| Feed : | 8-10 ml/min |

Im Abscheidefilter des Sprühtrockners wurden Mikrokristalle mit folgender Korngrößenverteilung erhalten

| | Partikelgröße(µm |
|---|---|
| X10 | 1,75 |
| X50 | 6,04 |
| X90 | 13 |
| X100 | 36 |

0,13% Wasser
0,12% Ethylacetat

### Beispiel 3

**Herstellung von Kapseln mit mikrokristallinem Carbamat J956:**

| **Substanz** | **Menge** |
|---|---|
| J956, mikrokristallin | 1,000 mg |
| Mikrokristalline Cellulose | 102,480 mg |
| Magnesiumstearat | 0,520 mg |
| Hartgelatinekapsel, Größe 3 | 1 Stück |
| Kapselfüllmasse | 104,000 mg |

Das mikrokristalline J956 wird in einem geeignetem Mischer (z.B. Containermischer) mit der mikrokristallinen Cellulose gemischt. Das Magnesiumstearat wir zugegeben und nochmals gemischt. Die Abwesenheit von Wasser in den Geräten ist zu prüfen.
Die Mischung wird mit einer geeigneten Kapselfüllmaschine (z.B. Harro Höflinger, KFMIIIC) in Hartgelatinekapseln Größe 3 abgefüllt.

### Beispiel 4

**Herstellung von überzogenen Tabletten mit mikrokristallinem Carbamat J956**

| **Kern:** | |
|---|---|
| J956, mikrokristallin | 1,00 mg |
| Laktose-Monohydrat | 33,8 mg |
| Maisstärke | 18,0 mg |
| Maltodextrin (10 % in Wasser) | 6,0 mg |
| Na-Carboxymethylstärke | 0,6 mg |
| Glycerol-Monobehenat | 0,6 mg |

| **Hülle:** | |
|---|---|
| Hydroxypropylmethylcellulose | 1.125 mg |
| Talkum | 0.225 mg |
| Titandioxid | 0.625 mg |
| Eisenoxid, Pigment gelb | 0.020 mg |
| Eisenoxid, Pigment rot | 0.005 mg |

Das mikrokristalline J956 wird in einem Granulator (z.B. Wirbelschichtgranulator GPCG 3.1, Fa. Glatt) mit Laktose und Maisstärke gemischt. Auf die Mischung wird eine Lösung von Maltodextrin in Wasser aufgesprüht und das entstehende Granulat wird getrocknet (Zuluftftemperatur 70 °C). Das Granulat wird mit Na-Carboxymethylstärke und Glycerol-Monobehenat gemischt und zu Tablettenkemen der Masse 150 mg verpresst. Die Tablettenkerne werden in einem geeignetem Coater (z.B. Driacoater 500, Driam) mit einer Suspension der Hüllensubstanzen in wasser besprüht und der entstehende Film getrocknet (Filmmasse 4 mg, Zulufttemperatur 70 °C, Trocknungsverlust der Filmtablette 3%).

## Patentansprüche

1. Verfahren zum Herstellen von Kristallen, deren durchschnittliche Partikelgröße 3 µm bis 25 µm beträgt und deren maximale Partikelgrößen 100 µm nicht überschreiten, wobei eine übersättigte Lösung von 11β-Benzaldoxim-estra-4,9-dien der Formel (1) 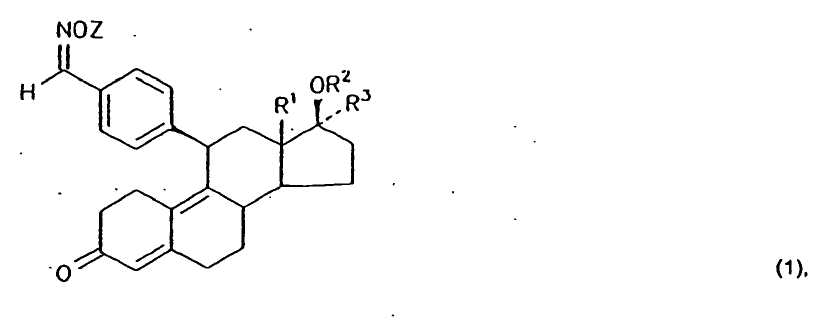
R¹ ein Wasserstoffatom oder ein Alkylrest mit 1-6 Kohlenstoffatomen ist,
R² für ein Wasserstoffatom, eine Alkyl-, Aryl-, Aralkyl- oder Alkylarylgruppe mit 1-10 Kohlenstoffatomen, einen Acylrest mit 1-10 Kohlenstoffatomen oder einen Rest -CONHR⁴ oder -COOR⁴ steht, wobei R⁴ ein Wasserstoffatom, einen Alkyl-, Aryl-, Aralkyl- oder Alkylarylrest mit 1-10 Kohlenstoffatomen bedeutet,
R³ ein Wasserstoffatom, eine Alkyl-, Aryl-, Aralkyl- oder Alkylarylgruppe mit 1-10 Kohlenstoffatomen, einen Rest -(CH₂)ₙ-CH₂X, wobei n=0, 1 oder 2 ist, X für ein Wasserstoffatom, für einen Alkyl-, Aryl-, Aralkyl- oder Alkylarylrest mit 1-10 Kohlenstoffatomen, für ein Fluor-, Chlor-, Brom- oder lodatom, für eine Cyano-, Azido- oder Rhodanogruppe, für einen Rest OR⁵ oder SR⁵ steht, wobei R⁵ ein Wasserstoffatom, ein Alkyl-, Aryl-, Aralkyl- oder Alkylarylrest mit 1-10 Kohlenstoffatomen oder ein Acylrest mit 1-10 Kohlenstoffatomen ist.
einen Rest OR⁵, wobei R⁵ die oben angegebene Bedeutung hat,
einen Rest -(CH₂)ₒ-CH=CH(CH₂)ₚ-R⁶, wobei o=0, 1, 2 oder 3 und p=0, 1 oder 2 ist und R⁶ für ein Wasserstoffatom, eine Alkyl-, Aryl-, Aralkyl- oder Alkylarylgruppe mit 1-10 Kohlenstoffatomen, eine Hydroxylgruppe, eine Alkoxygruppe oder Acyloxygruppe mit 1-10 Kohlenstoffatomen steht,
einen Rest -(CH₂)_{q}C=CR⁷, wobei q=0, 1 oder 2 und R⁷ ein Wasserstoffatom, ein Fluor-, Chlor-, Brom- oder lodatom, ein Alkyl-, Aryl-, Aralkyl- oder Alkylarylrest mit 1-10 Kohlenstoffatomen oder ein Acylrest mit 1-10 Kohlenstoffatomen ist, bedeutet,
Z für ein Wasserstoffatom, einen Alkyl-, Aryl-, Aralkyl- oder Alkylarylrest mit 1-10 Kohlenstoffatomen, einen Acylrest mit 1-10 Kohlenstoffatomen, einen Rest -CONHR⁴ oder -COOR⁴, wobei R⁴ ein Wasserstoffatom, einen Alkyl-, Aryl-, Aralkyl- oder Alkylarylrest mit 1-10 Kohlenstoffatomen bedeutet,
oder für ein Alkali- oder Erdalkalimetallatom steht, sowie deren pharmazeutisch annehmbaren Salze,
während der Kristallisation einem Naßmahlen mittels einer Vorrichtung zum Naßmahlen unterzogen wird, wodurch eine Primärkornsuspension erhalten wird.

2. Verfahren nach Anspruch 1, wobei das 11 β-Benzaldoxim-estra-4,9-dien die Verbindung 11β-{4-[(Ethylaminocarbonyl)oximinomethyl]phenyl}-17β-methoxy-17α-methoxymethyl-estra-4,9-dien-3-on ist.

3. Verfahren nach einem der vorhergehenden Ansprüche, wobei die übersättigte Lösung 10 Gew.-% bis 30 Gew.-%, bezogen auf die übersättigte Lösung, der Verbindung der Formel (1) in einem Lösungsmittel enthält.

4. Verfahren nach Anspruch 3, wobei das Lösungsmittel Ethylacetat ist.

5. Verfahren nach einem der vorhergehenden Ansprüche, wobei die übersättigte Lösung hergestellt wird durch Auflösen einer Verbindung der Formel (1) in einem Lösungsmittel bei einer Temperatur unterhalb des Siedepunkts des Lösungsmittels und nachfolgendem Abkühlen auf eine Temperatur oberhalb des Gefrierpunkts der Lösung.

6. Verfahren nach einem der vorhergehenden Ansprüche, wobei die Kristallisation in einem einen Rührer aufweisenden Gefäß durchgeführt wird.

7. Verfahren nach einem der vorhergehenden Ansprüche, wobei die Vorrichtung zum Naßmahlen ein Rotor-Stator-Werkzeug, eine Rührwerksmühle, ein Walzenstuhl oder eine Kolloidmühle ist.

8. Verfahren nach einem der vorhergehenden Ansprüche, wobei die Primärkornsuspension auf eine Temperatur Tₘₐₓ unterhalb der Löslichkeitsgrenze der Primärkömer in der Suspension erwärmt und nachfolgend auf eine Temperatur Tₘᵢₙ oberhalb des Gefrierpunkts der Suspension abkühlt wird.

9. Verfahren nach Anspruch 8, wobei Tₘₐₓ so gewählt wird, das 10 Gew.-% bis 90 Gew.-% der Primärkömer im Lösungsmittel aufgelöst werden.

10. Verfahren nach einem der Ansprüche 8 oder 9, wobei Tₘᵢₙ so gewählt wird, das die aufgelösten Primärkörner im wesentlichen wieder kristallisieren.

11. Verfahren nach einem der Ansprüche 8 bis 10, wobei das Abkühlen von Tₘₐₓ auf Tₘᵢₙ während 1 Minute bis 10 Std. erfolgt.

12. Verfahren nach einem der Ansprüche 8 bis 11, wobei das Erwärmen auf Tₘₐₓ und das Abkühlen auf Tₘᵢₙ 1 bis 10 mal durchgeführt wird.

13. Kristalle von 11β-Benzaldoxim-estra-4,9-dien der Formel (1), erhältlich nach einem Verfahren gemäß einem der Ansprüche 1 bis 12.

14. Pharmazeutische Formulierung, enthaltend Kristalle von 11β-Benzaldoxim-estra-4,9-dien der Formel (1) nach Anspruch 13.

## Claims

1. Process for the preparation of crystals whose mean particle size is 3 µm to 25 µm and whose maximum particle sizes do not exceed 100 µm, wherein a supersaturated solution of 11β-benzaldoximoestra-4,9-diene of formula (1): 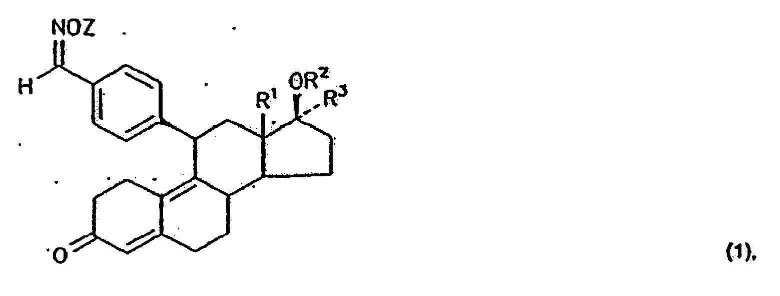 in which:
R¹ is a hydrogen atom or an alkyl radical having 1-6 carbon atoms,
R² is a hydrogen atom, an alkyl, aryl, aralkyl or alkylaryl group having 1-10 carbon atoms, an acyl radical having 1-10 carbon atoms or a radical -CONHR⁴ or -COOR⁴, R⁴ being a hydrogen atom or an alkyl, aryl, aralkyl or alkylaryl radical having 1-10 carbon atoms,
R³ is a hydrogen atom, an alkyl, aryl, aralkyl or alkylaryl group having 1-10 carbon atoms, a radical -(CH₂)n-CH₂X, n being 0, 1 or 2 and X being a hydrogen atom, an alkyl, aryl, aralkyl or alkylaryl radical having 1-10 carbon atoms, a fluorine, chlorine, bromine or iodine atom, a cyano, azido or rhodano group or a radical OR⁵ or SR⁵, R⁵ being a hydrogen atom, an alkyl, aryl, aralkyl or alkylaryl radical having 1-10 carbon atoms or an acyl radical having 1-10 carbon atoms,
a radical OR⁵, R⁵ being as defined above,
a radical -(CH₂)ₒ-CH=CH(CH₂)ₚ-R⁶, o being 0, 1, 2 or 3, p being 0, 1 or 2 and R⁶ being a hydrogen atom, an alkyl, aryl, aralkyl or alkylaryl group having 1-10 carbon atoms, a hydroxyl group or an alkoxy group or acyloxy group having 1-10 carbon atoms, or
a radical -(CH₂)_{q}C=CR⁷, q being 0, 1 or 2 and R⁷ being a hydrogen atom, a fluorine, chlorine, bromine or iodine atom, an alkyl, aryl, aralkyl or alkylaryl radical having 1-10 carbon atoms or an acyl radical having 1-10 carbon atoms, and
Z is a hydrogen atom, an alkyl, aryl, aralkyl or alkylaryl radical having 1-10 carbon atoms, an acyl radical having 1-10 carbon atoms, a radical -CONHR⁴ or -COOR⁴, R⁴ being a hydrogen atom or an alkyl, aryl, aralkyl or alkylaryl radical having 1-10 carbon atoms,
or an alkali metal or alkaline earth metal atom, and their pharmaceutically acceptable salts,
is subjected during crystallization to wet grinding by means of a wet grinding device to give a primary particle suspension.

2. Process according to Claim 1 wherein the 11β-benzaldoximoestra-4,9-diene is the compound 11β-{4-[(ethylarninocarbonyl)oximinomethyl]phenyl}-17β-methoxy-17α-methoxymethyloestra-4,9-dien-3-one.

3. Process according to one of the preceding claims wherein the supersaturated solution contains 10% by weight to 30% by weight, based on the supersaturated solution, of the compound of formula (1) in a solvent.

4. Process according to Claim 3 wherein the solvent is ethyl acetate.

5. Process according to one of the preceding claims wherein the supersaturated solution is prepared by dissolving a compound of formula (1) in a solvent at a temperature below the boiling point of the solvent, and then cooling to a temperature above the freezing point of the solution.

6. Process according to one of the preceding claims wherein the crystallization is carried out in a vessel having a stirrer.

7. Process according to one of the preceding claims wherein the wet grinding device is a rotor-stator appliance, an agitating mill, a cylinder mill or a colloid mill.

8. Process according to one of the preceding claims wherein the primary particle suspension is heated to a temperature Tₘₐₓ below the solubility limit of the primary particles in the suspension, and then cooled to a temperature Tₘᵢₙ above the freezing point of the suspension.

9. Process according to Claim 8 wherein Tₘₐₓ is chosen so that 10% by weight to 90% by weight of the primary particles are dissolved in the solvent.

10. Process according to Claim 8 or 9 wherein Tₘᵢₙ is chosen so that the dissolved primary particles substantially crystallize again.

11. Process according to one of Claims 8 to 10 wherein the cooling from Tₘₐₓ to Tₘᵢₙ takes place over 1 minute to 10 hours.

12. Process according to one of Claims 8 to 11 wherein the heating to Tₘₐₓ and the cooling to Tₘᵢₙ are carried out 1 to 10 times.

13. Crystals of 11β-benzaldoximoestra-4,9-diene of formula (1) obtainable by a process according to one of Claims 1 to 12.

14. Pharmaceutical formulation containing crystals of 11β-benzaldoximoestra-4,9-diene of formula (1) according to Claim 13.

## Revendications

1. Procédé de préparation de cristaux dont la taille de particules moyenne est de 3 µm à 25 µm et dont les tailles de particules maximales ne dépassent pas 100 µm, où une solution sursaturée de 11β-benzaldoxime-oestra-4,9-diène de formule (1) 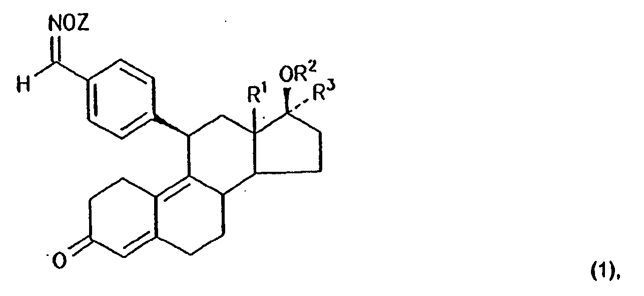 dans laquelle
R¹ représente un atome d'hydrogène ou un radical alkyle de 1 à 6 atomes de carbone,
R² représente un atome d'hydrogène, un groupe alkyle, aryle, aralkyle ou alkylaryle de 1 à 10 atomes de carbone, un radical acyle de 1 à 10 atomes de carbone ou un radical -CONHR⁴ ou -COOR⁴, où R⁴ représente un atome d'hydrogène, un radical alkyle, aryle, aralkyle ou alkylaryle de 1 à 10 atomes de carbone,
R³ représente un atome d'hydrogène, un groupe alkyle, aryle, aralkyle ou alkylaryle de 1 à 10 atomes de carbone, un radical -(CH₂)ₙ-CH₂X, où n=0, 1 ou 2, X représente un atome d'hydrogène, un radical alkyle, aryle, aralkyle ou alkylaryle de 1 à 10 atomes de carbone, un atome de fluor, de chlore, de brome ou d'iode, un groupe cyano, azido ou rhodano, un radical OR⁵ ou SR⁵, où R⁵ est un atome d'hydrogène, un radical alkyle, aryle, aralkyle ou alkylaryle de 1 à 10 atomes de carbone ou un radical acyle de 1 à 10 atomes de carbone,
un radical OR⁵, où R⁵ a la signification susmentionnée,
un radical -(CH₂)ₒ-CH=CH(CH₂)ₚ-R⁶, où o=0, 1, 2 ou 3 et p=0, 1 ou 2 et R⁶ représente un atome d'hydrogène, un groupe alkyle, aryle, aralkyle ou alkylaryle de 1 à 10 atomes de carbone, un groupe hydroxyle ou un groupe acyloxy de 1 à 10 atomes de carbone,
un radical -(CH₂)_{q}-C=CR⁷, où q=0, 1 ou 2 et R⁷ représente un atome d'hydrogène, un atome de fluor, de chlore, de brome ou d'iode, un radical alkyle, aryle, aralkyle ou alkylaryle de 1 à 10 atomes de carbone ou un radical acyle de 1 à 10 atomes de carbone,
Z représente un atome d'hydrogène, un radical alkyle, aryle, aralkyle ou alkylaryle de 1 à 10 atomes de carbone, un radical acyle de 1 à 10 atomes de carbone, un radical -CONHR⁴ ou -COOR⁴, où R⁴ représente un atome d'hydrogène, un radical alkyle, aryle, aralkyle ou alkylaryle de 1 à 10 atomes de carbone,
ou un atome de métal alcalin ou alcalino-terreux ainsi que ses sels pharmaceutiquement acceptables,
est soumises à un broyage à l'état humide pendant la cristallisation au moyen d'un dispositif destiné au broyage par voie humide, moyennant quoi on obtient une suspension de grains primaires.

2. Procédé selon la revendication 1, où le 11β-benzaldoxime-oestra-4,9-diène est le composé 11β-(4-[{éthylaminocarbonyl}oximinométhyl]-phényl)-17β-méthoxy-17α-méthoxyméthyl-oestra-4,9-dièn-3-one.

3. Procédé selon l'une des revendications précédentes, où la solution sursaturée comprend 10 % en poids à 30 % en poids, par rapport à la solution sursaturée, du composé de formule (1) dans un solvant.

4. Procédé selon la revendication 3, où le solvant est l'acétate d'éthyle.

5. Procédé selon l'une des revendications précédentes, où la solution sursaturée est préparée par dissolution d'un composé de formule (1) dans un solvant à une température située en dessous du point d'ébullition du solvant et par refroidissement consécutif à une température située au-dessus du point de congélation de la solution.

6. Procédé selon l'une des revendications précédentes, où la cristallisation est réalisée dans un récipient muni d'un agitateur.

7. Procédé selon l'une des revendications précédentes, où le dispositif destiné au broyage par voie humide est un outil à rotor - stator, un broyeur agitateur, un moulin à cylindres ou un broyeur colloïdal.

8. Procédé selon l'une des revendications précédentes, où la suspension de grains primaires est chauffée à une température Tₘₐₓ située en dessous du seuil de solubilité des grains primaires dans la suspension et est ensuite refroidie à une température Tₘᵢₙ située au-dessus du point de congélation de la suspension.

9. Procédé selon la revendication 8, où Tₘₐₓ est choisie pour que 10 % en poids à 90 % en poids des grains primaires soient dissous dans le solvant.

10. Procédé selon l'une des revendications 8 ou 9, où Tₘᵢₙ est choisie pour que les grains primaires dissous se recristallisent sensiblement.

11. Procédé selon l'une des revendications 8 à 10, où le refroidissement de Tₘₐₓ à Tₘᵢₙ s'effectue en 1 minute à 10 heures.

12. Procédé selon l'une des revendications 8 à 11, où le chauffage à Tₘₐₓ et le refroidissement à Tₘᵢₙ est réalisé de 1 à 10 fois.

13. Cristaux de 11β-benzaldoxime-oestra-4,9-diène de formule (1), pouvant être obtenus d'après un procédé selon l'une des revendications 1 à 12.

14. Formulation pharmaceutique contenant des cristaux de 11β-benzaldoxime-oestra-4,9-diène de formule (1) selon la revendication 13.
